# EUROPEAN PATENT APPLICATION

(11) **EP 4 563 148 A1**
(43) Date of publication of application: **04.06.2025**
(21) Application number: 23846653.6
(22) Date of filing: 28.07.2023
(51) Int. Cl.: A61K 31/455, A61P 35/00, A61P 39/00, A61P 39/06, A61P 43/00, C12N 9/10, C12N 15/09

(54) **MEDICINE FOR TREATING CANCER**

(30) Priority: 28.07.2022 JP 2022121024
(71) Applicant: Science Technology Interact Co., Ltd., Tokyo 103-0025 (JP); Tohoku University, Sendai-shi, Miyagi 980-8577 (JP)
(72) Inventor: ICHIMIYA, Kazuko, Tokyo 103-0025 (JP); CHIBA, Natsuko, Sendai-shi, Miyagi 980-8577 (JP); JINGU, Keiichi, Sendai-shi, Miyagi 980-8577 (JP); YOSHINO, Yuki, Sendai-shi, Miyagi 980-8577 (JP); FUJITA, Yuhzo, DECEASED (JP)
(74) Representative: Winter, Brandl - Partnerschaft mbB
(86) International application number: PCT/JP2023/027683
(87) International publication number: WO 2024/024923

(57) **Abstract**

An object of the present invention is to provide a medicine for promoting DNA damage caused by irradiation in malignant tumor (cancer) cells while suppressing DNA damage caused by irradiation in normal cells. The present invention provides a medicine for promoting DNA damage caused by irradiation in malignant tumor (cancer) cells while suppressing DNA damage caused by irradiation in normal cells, the medicine containing Nicaraven as an active ingredient.

## Description

### Technical Field

The present invention relates to a medicine for treating cancer.

### Background Art

Radiation therapy, in which malignant tumor (cancer) cells are suppressed from proliferating or killed by DNA damage, is one of effective options for cancer treatment. However, since radiation therapy damages not only DNA of malignant tumor (cancer) cells but also DNA of normal cells, studies on radioprotectants are also in progress. Specifically, development of a radioprotectant capable of reducing side effects caused by irradiation by elimination of radicals generated by an indirect action of irradiation, hydrogen addition, reduction of an oxygen effect, and the like has been advanced.

However, on the other hand, there is a concern that the radioprotective effect attenuates the therapeutic effect of irradiation on malignant tumor (cancer) cells in the first place. Accordingly, development of a drug that promotes the therapeutic effect of irradiation and suppresses side effects is desired.

### Citation List

### Patent Literature

PTL 1: JPH06-145057A

### Non-patent Literature

NPL 1: Watanabe M, Akiyama N, Sekine H, et al.: Inhibition of poly (ADP-ribose) polymerase as a protective effect of nicaraven in ionizing radiation- and ara-C-induced cell death. Anticancer Res, 2006; 26. 3421-3428
NPL 2: Yoshino Y, Endo S, Chen Z, et al.: Evaluation of site-specific homologous recombination activity of BRCA1 by direct quantitation of gene editing efficiency. Sci Rep, 2019; 9. 1644
NPL 3: Yong Xu, Da Zhai, Shinji Goto, Xu Zhang, Keiichi Jingu and Tao-Sheng Li, Nicaraven mitigates radiation-induced lung injury by downregulating the NF-κB and TGF-β/Smad pathways to suppress the inflammatory response. J RadRes, 202263(2). 158-165

### Summary of Invention

### Technical Problem

An object of the present invention is to provide a medicine for promoting DNA damage caused by irradiation in malignant tumor (cancer) cells while suppressing DNA damage caused by irradiation in normal cells.

### Solution to Problem

Under such circumstances, as a result of intensive studies, the present inventors have found that, among a wide variety of compounds, Nicaraven has both radical scavenger activity and PARP inhibitory activity. The present inventors have obtained findings that, with regard to Nicaraven, a radiosensitizing effect in which DNA damage caused by irradiation accumulates due to a PARP inhibitory effect is exhibited in cells having a fast cell cycle (tumor cells), and a function as a radical scavenger is more enhanced in cells having a slow cell cycle (normal cells), so that an effect of suppressing DNA damage caused by irradiation in normal cells while promoting DNA damage caused by irradiation in malignant tumor cells can be compatibly achieved. The present invention is based on such novel findings. Accordingly, the present invention provides the following items:
Item 1. A medicine having both radical scavenger activity and PARP inhibitory activity, including
   Nicaraven as an active ingredient.
Item 2. A medicine for use in suppression of DNA damage caused by irradiation in normal cells by a radical scavenger function and inhibition of repair of DNA damage in malignant tumor (cancer) cells by a PARP suppressive action, including Nicaraven as an active ingredient.
Item 3. The medicine according to item 1 or 2, for use in treatment of cancer in combination with irradiation.
Item 4. The medicine according to item 3, being administered to a subject prior to the irradiation.
Item 5. A method for treating cancer by suppressing DNA damage caused by irradiation in normal cells by a radical scavenger function and inhibiting repair of DNA damage in malignant tumor (cancer) cells by a PARP suppressive action, including
   administering to a subject in need of the treatment of cancer an effective amount of Nicaraven.
Item 6. The method according to item 5, including irradiating the subject.
Item 7. The method according to item 6, wherein the Nicaraven is administered to the subject prior to the irradiation.
Item 8. Nicaraven for use in treatment of cancer by suppressing DNA damage caused by irradiation in normal cells by a radical scavenger function and inhibiting repair of DNA damage in malignant tumor (cancer) cells by a PARP suppressive action.
Item 9. The Nicaraven for use according to item 8, wherein the treatment of cancer is performed in combination with irradiation.
Item 10. The Nicaraven for use according to item 9, being administered to a subject prior to the irradiation.
Item 11. A pharmaceutical composition for use in treatment of cancer by suppressing DNA damage caused by irradiation in normal cells by a radical scavenger function and inhibiting repair of DNA damage in malignant tumor (cancer) cells by a PARP suppressive action, including
   Nicaraven and a pharmaceutical carrier.
Item 12. The pharmaceutical composition for use according to item 11, wherein the treatment of cancer is performed in combination with irradiation.
Item 13. The pharmaceutical composition for use according to item 12, wherein the Nicaraven is administered to a subject prior to the irradiation.
Item 14. Use of Nicaraven for producing an agent for treating cancer by suppressing DNA damage caused by irradiation in normal cells by a radical scavenger function and inhibiting repair of DNA damage in malignant tumor (cancer) cells by a PARP suppressive action.
Item 15. The use according to item 14, wherein the agent is used for treating cancer in combination with irradiation.
Item 16. The use according to item 15, wherein Nicaraven is administered to a subject prior to the irradiation.

### Advantageous Effects of Invention

The medicine of the present invention can compatibly achieve an effect of promoting DNA damage caused by irradiation in malignant tumor (cancer) cells while suppressing DNA damage caused by irradiation in normal cells.

Conventionally, a report that Nicaraven is used for radioprotection (PTL 1) and a report that Nicaraven has PARP inhibitory activity involved in cell death (NPL 1) have been made separately. However, in general, reduction of side effects caused by irradiation and promotion of cell death are opposite effects to each other. Therefore, it has been expected that Nicaraven reduces side effects caused by irradiation, but also suppresses therapeutic effects caused by irradiation. However, as previously described, the inventors have shown that Nicaraven can surprisingly compatibly achieve PARP inhibitory action in addition to radical scavenger action. Specifically, the present inventors have found that a PARP inhibitory action is more generated in cells having a fast cell cycle (tumor cells), and a function as a radical scavenger is more strongly exerted in cells having a slow cell cycle (normal cells), and thus, a seemingly contradictory effect of promoting DNA damage caused by irradiation in tumor cells while suppressing DNA damage caused by irradiation in normal cells is compatibly achieved.

Hitherto, there has been no compound which is expected to exhibit a PARP inhibitory action on tumor cells and have a function of reducing side effects of irradiation as a radical scavenger on normal cells by one drug. Therefore, such an effect of the present invention cannot be expected from the prior art.

In the present invention, although not all the mechanisms by which Nicaraven promotes DNA damage caused by irradiation in tumor cells while suppressing DNA damage caused by irradiation in normal cells have been clarified, the following is considered:
1. Nicaraven inhibits repair of DNA single-strand break (SSB) by the inhibitory action of PARP, which functions in DNA single-strand break repair. SSB is converted into DNA double-strand break (DSB) that is more severe DNA damage, and the like during DNA replication and the like associated with progression of the cell cycle. Inhibition of PARP is not fatal because DSB is repaired by homologous recombination (HR) repair in normal cells. On the other hand, in malignant tumor (cancer) cells with impaired HR repair, DSB accumulates without being repaired, so that apoptosis is caused to kill the cells.

In addition, Nicaraven suppresses PARP required for the transcriptional action of NF-κB, and suppresses BCL-2, a suppressor of apoptosis, which is downstream of transcription of NF-κB, thereby inducing apoptosis.
2. As described in 1., since SSB accumulated due to PARP inhibition is converted into DSB at the time of DNA replication and the like, DSB is more generated due to PARP inhibition in tumor cells in which the cell cycle is actively progressing. On the other hand, it is considered that the radical scavenger activity of Nicaraven is not affected by the cell cycle. Therefore, it is considered that Nicaraven exhibits a protective effect against irradiation in normal cells in which the progress of the cell cycle is slow, and converts SSB generated by irradiation into more severe DSB by a PARP inhibitory effect in malignant tumor (cancer) cells in which the cell cycle is fast and the HR repair activity is abnormal, thereby exhibiting a sensitizing effect of irradiation.
3. Irradiation activates NF-κB. In addition, in malignant tumors, NF-κB is originally elevated, and the activity of the NFκB-heparanase pathway is increased, so that adhesion between cells is lost and metastasis is facilitated. Nicaraven suppresses the activity of NF-κB, thereby suppressing its downstream heparinase enzyme activity, so that metastasis is suppressed.
4. On the other hand, induction and release of an inflammation signal (TNF-α, IL-6) are suppressed by suppressing the NF-κB-heparanase activity in the same pathway as in 3, and inflammation caused by an anticancer agent and irradiation is suppressed.

### Brief Description of Drawings

Fig. 1 illustrates an experimental protocol and experimental results of a test for PARP inhibitory activity of Nicaraven in Example 1.
Fig. 2 illustrates an experimental protocol and experimental results of a test for the effect of Nicaraven treatment on HR activity in Example 2.
Fig. 3 illustrates an experimental protocol and experimental results of a test for the effect of Nicaraven treatment on DNA double-strand break in the DNA repair process in Example 3.
Fig. 4 illustrates an experimental protocol and experimental results of a test for the effect of Nicaraven treatment on DNA double-strand break caused by IR in Example 4.

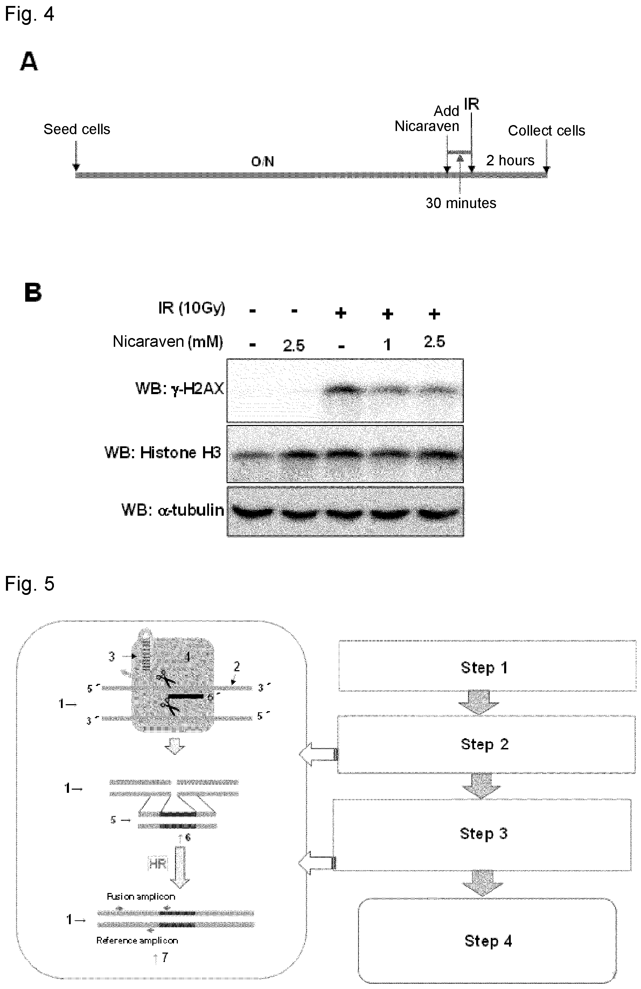

Fig. 5 illustrates an outline of a method for measuring homologous recombination repair (HR) activity (Assay for site-specific HR activity (ASHRA)) performed in Examples. In this method, a CRISPR/Cas9 system used for genome editing as a site-specific DNA cleaving enzyme is used to create a site-specific DNA double strand break in a target endogenous gene. Then, a fusion gene of a reporter and a target gene is generated by HR between a DNA fragment as a substrate for HR containing the reporter and the target endogenous gene. The production efficiency of the fusion gene of a reporter and a target gene is quantified as the HR repair activity of cell in the cell. For the method for detecting a fusion gene, flow cytometry, Western blotting, quantitative PCR, or the like can be used while changing the reporter. Step 1: Introduce Cas9/guide RNA expression vector and donor vector into cells. Step 2: Create a DNA double strand break specifically in target gene by Cas9/gRNA complex. Step 3: Repair DNA double strand break with HR using donor vector as template, whereby marker sequence is knocked in. Step 4: Detecting knock-in efficiency of marker ((1) Western blotting, (2) flow cytometry, (3) quantitative PCR). 1. Genomic DNA. 2. Target gene. 3. Guide RNA. 4. cas9. 5. Donor vector. 6. Marker sequence. 7. Fusion gene of marker and target gene
Fig. 6 illustrates an experimental protocol and experimental results of a test for PARP inhibitory activity of Nicaraven in Example 5.
Fig. 7 illustrates an experimental protocol and experimental results of a test for PARP inhibitory activity of Nicaraven in Example 5.

### Description of Embodiments

### Medicine for Promoting DNA Damage Caused by Irradiation in Malignant Tumor (Cancer) Cells while Suppressing DNA Damage Caused by Irradiation in Normal Cells

The present invention provides a medicine for promoting DNA damage caused by irradiation in malignant tumor (cancer) cells while suppressing DNA damage caused by irradiation in normal cells, the medicine containing Nicaraven as an active ingredient.

Examples of the cancer to be treated with the medicine of the present invention include skin cancer, mesothelioma, lung cancer, stomach cancer, liver cancer, large bowel cancer, breast cancer, esophageal cancer, pancreatic cancer, uterine cancer (cervical cancer, endometrial cancer, etc.), ovarian cancer, skin cancer, urinary cancer, head and neck cancer, hematopoietic tumor (leukemia, lymphoma, etc.), and bone and soft tissue sarcoma.

In the present invention, Nicaraven itself may be used as a medicine, or may be used as a pharmaceutical composition in combination with various pharmaceutically acceptable carriers (for example, tonicity agents, chelating agents, stabilizing agents, pH adjusting agents, preservatives, antioxidants, solubilizing agents, thickening agents, and the like).

Examples of the tonicity agent include saccharides such as glucose, trehalose, lactose, fructose, mannitol, xylitol, and sorbitol, polyhydric alcohols such as glycerin, polyethylene glycol, and propylene glycol, and inorganic salts such as sodium chloride, potassium chloride, and calcium chloride.

Examples of the chelating agent include edetates such as disodium edetate, calcium disodium edetate, trisodium edetate, tetrasodium edetate, and calcium edetate, ethylenediamine tetraacetate, nitrilotriacetic acid or a salt thereof, sodium hexametaphosphate, and citric acid.

Examples of the stabilizing agent include sodium bisulfite.

Examples of the pH adjusting agent include acids such as hydrochloric acid, carbonic acid, acetic acid, and citric acid, and further include alkali metal hydroxides such as sodium hydroxide and potassium hydroxide, alkali metal carbonates or hydrogen carbonates such as sodium carbonate, alkali metal acetates such as sodium acetate, alkali metal citrates such as sodium citrate, and bases such as trometamol.

Examples of the preservative include paraoxybenzoates such as sorbic acid, potassium sorbate, methyl paraoxybenzoate, ethyl paraoxybenzoate, propyl paraoxybenzoate, and butyl paraoxybenzoate; quaternary ammonium salts such as chlorhexidine gluconate, benzalkonium chloride, benzethonium chloride, and cetylpyridinium chloride; alkylpolyaminoethylglycine, chlorobutanol, polyquad, polyhexamethylene biguanide, and chlorhexidine.

Examples of the antioxidant include sodium bisulfite, dry sodium sulfite, sodium pyrosulfite, and concentrated mixed tocopherol.

Examples of the solubilizing agent include sodium benzoate, glycerin, D-sorbitol, glucose, propylene glycol, hydroxypropylmethyl cellulose, polyvinylpyrrolidone, macrogol, and D-mannitol. Examples of the thickening agent include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methyl cellulose, polyvinyl pyrrolidone, and polyvinyl alcohol.

In addition, the pharmaceutical composition may further contain, in addition to Nicaraven, a compound known to have an antitumor effect.

In the embodiment of the pharmaceutical composition, the content of Nicaraven in the composition is not particularly limited, and can be appropriately set from conditions of, for example, 90 mass% or more, 70 mass% or more, 50 mass% or more, 30 mass% or more, 10 mass% or more, 5 mass% or more, and 1 mass% or more.

The formulation form is not particularly limited, and examples thereof include various formulation forms such as: oral administration agents in forms of tablets, pills, capsules, powders, granules, syrups, oral rinses, drops, and sublingual agents etc.; injections (intravenous injection, intramuscular injection, local injection, intraperitoneal injection, etc.); external preparations (ointments, creams, patches, inhalants); and parenteral administration agents like suppositories. Preferred examples of the formulation form include oral administration agents and injections.

The content of the Nicaraven of the present invention in the formulation varies depending on the administration route, the age, body weight and symptoms of the patient, and the like, and thus cannot be unconditionally defined, but the daily dose of the compound of the present invention may be adjusted to an amount that is usually about 10 to 5000 mg, more preferably about 100 to 1000 mg. In the case of administration once a day, this amount may be contained in one formulation, and in the case of administration three times a day, this 1/3 amount may be contained in one formulation.

The medicine of the present invention is administered to a patient such as a mammal. Examples of the mammal include human, monkey, mouse, rat, rabbit, cat, dog, pig, cow, horse, and sheep.

### PARP Inhibitor for Inhibiting PARP in Malignant Tumor (Cancer) Cells while Suppressing DNA Damage Caused by Irradiation in Normal Cells

The medicine of the present invention causes a sensitizing effect of irradiation due to accumulation of DNA damage caused by irradiation by inhibiting PARP in malignant tumor (cancer) cells while suppressing DNA damage in normal cells.

Accordingly, in another embodiment, the present invention provides a PARP inhibitor containing Nicaraven as an active ingredient for inhibiting PARP in malignant tumor (cancer) cells while suppressing DNA damage caused by irradiation in normal cells. Poly(ADP-ribose)polymerase (PARP) is a family of proteins involved in many cellular processes such as DNA repair, genomic stability, and programmed cell death. For example, PAR polymerase 1 (PARP1) binds to a damaged portion of DNA and repairs DNA damage by Poly(ADP-ribosyl)ation (adding poly(ADP-ribose)) using NAD as a substrate. Therefore, the PARP inhibitor of the present invention can suppress proliferation of malignant tumor (cancer) cells or kill malignant tumor (cancer) cells by inhibiting PARP. The active ingredient, formulation form, dose, and the like of the PARP inhibitor are the same as those of the medicine of the present invention.

Hereinafter, specific embodiments of the present invention will be exemplarily described in detail by using Examples, but the present invention is not limited to such Examples.

### Examples

### Test Method

### Cell Culture

HeLa cells (human cervical cancer cell line) were purchased from ATCC (Manassas, VA, USA). HeLa cells were cultured in DMEM medium (Nissui Pharmaceutical Co., Ltd., Tokyo, Japan) supplemented with 8% Fetal Bovine Serum (FBS) (Biowest Inc., Nuaille, France) and 3% L-glutamine at 37°C in the presence of 5% CO2.

KYSE270 cells and KYSE30 cells (human esophageal squamous cell carcinoma cell line) were purchased from JCRB Cell Bank, National Institutes of Biomedical Innovation, Health and Nutrition (Osaka, Japan). KYSE270 cells and KYSE30 cells were cultured in a medium in which RPMI1640 and Ham's F12 medium (both of which were manufactured by FUJIFILM Wako Pure Chemical Corporation, Osaka, Japan) were mixed in equal amounts and 5% FBS was added at 37°C in the presence of 5% CO2.

A stock solution of Nicaraven was prepared by dissolving Nicaraven in DMEM medium at a final concentration of 40 mM and sterilizing the solution through a filter. Olaparib (Adooq Bioscience, Irvine, CA, USA) was prepared as a stock solution of Olaparib in DMSO (FUJIFILM Wako Pure Chemical Corporation, Osaka, Japan) at a final concentration of 50 mM. Edaravone (FUJIFILM Wako Pure Chemical Corporation, Osaka, Japan) was prepared as a stock solution of Edaravone in DMSO (FUJIFILM Wako Pure Chemical Corporation, Osaka, Japan) at a final concentration of 100 mM.

### Plasmid DNA

As vectors expressing Cas9 and a guide RNA, LentiCRISPRv2-ACTB-C1 (Addgene ID: #169796) expressing a guide RNA targeting ACTB gene and LentiCRISPRv2-scr (Addgene ID: #169795) expressing a control guide RNA sequence were used, and as an HR donor vector for ACTB gene, pBS-ACTB-200 GFPfr1 (Addgene ID: #169798) was used.

### siRNA

siRNA of BRCA1 was produced as having a target sequence for 5'-GCUCCUCUCACUCUUCAGUTT-3', which was a 3'-UTR of BRCA1 gene (JBioS, Saitama, Japan), and used at 15 nM. siRNA of RAD51 (hs.Ri.RAD51.13.1) was purchased from Integrated DNA Technologies (Coralville, IA, USA), and the concentration was 5 nM for all uses. Control siRNA was produced using the SilencerTM negative control siRNA template set attached to Silencer (registered trademark) Select siRNA (Thermo Fischer Scientific, MO, USA).

### Gene Introduction

TransIT-X2 Dynamic Delivery System (Mirus Bio LLC, Madison, WI, USA) was used for introduction of siRNA into cells. PEI MAX (Polysciences, Inc, Warrington, PA, USA) was used for introduction of plasmid DNA into cells.

### Drug Sensitivity Test

HeLa cell suspension adjusted to 5000 cells/ml was seeded in a 96 well plate at 100 ul/well, and the next day, siRNA was introduced, followed by addition of Olaparib or Nicaraven. One hundred twenty hours after gene introduction and Olaparib addition, HeLa cells were replaced in a medium to which Presto Blue assay (Thermo-Fisher Scientific, Waltham, MA, USA) had been added so as to have a final concentration of 5%, followed by reaction in the presence of 5% CO2 at 37°C for 1 hour, and then fluorescence was measured under conditions of an excitation wavelength of 520 nm and a fluorescence wavelength of 580 to 640 nm using GloMax (registered trademark) Explorer Multimode Microplate Reader (Promega, Madison, WI, USA).

### Western Blot

The culture medium for the cells seeded on a 3.5 cm dish was removed, followed by washing with 1x PBS and then direct dissolution in a 1x Sodium dodecyl sulfate (SDS) sample buffer (2% SDS, 1% 2-Mercaptoethanol [2-ME], 50 mM Tris-HCL [pH 6.8], 12% glycerol, 1% Bromophenol blue [BPB]). Thereafter, the solution was subjected to ultrasonic treatment with Vibra cell (Sonics & Materials Inc., CT, USA) and heated at 95°C for 5 minutes to prepare a whole cell extract. For Western blotting, electrophoresis was performed through a 6%, 10% SDS polyacrylamide gel, and transcription was done to a PVDF membrane (Millipore Corporation Darmstadt, Germany) for 1.5 h, followed by blocking with 5% skim milk-PBS for 3 to 16 h. After blocking, a primary antibody diluted with a 0.5% BSA/TBS-T or Can get signal solution (TOYOBO, Osaka, Japan) was allowed to react overnight at 4°C. After washing 3 times with PBS-T for 10 minutes, a secondary antibody diluted with 0.5% BSA/TBS-T was allowed to react at room temperature for 1 to 3 hours. After washing 3 times for 10 minutes with PBS-T, detection was performed with LAS-4000 mini (GE healthcare, WI, USA) using Ez West Lumi plus (ATTO), Immuno (registered trademark) StarLD (FUJIFILM Wako Pure Chemical Co. Osaka, Japan), or an ECL solution (10 mM Tris-HCl [pH 8.8], 0.015% H2O2, 0.5 mM 4-(Imidazol-1-yl)phenol, and 0.625 mM luminol).

### Antibody

### Primary Antibodies

Anti-BRCA1 antibody polyclonal antibody x3000 produced using 1528-1863 amino acids as an antigen anti-RAD51 antibody N1C2, GTX100469 (Gene Tax, Irvine, CA, USA) x2000 anti-β-actin antibody 6D1 (Fujifilm Wako purechemicals) x2000 anti-γ-H2AX antibody JBW301 (Merck, Darmstadt, Germany), x1000 anti-HistonH3 antibody DAM 1776159 (Merck) x1000
anti-α-tubulin antibody DM1A (Merck) x5000

As a secondary antibody, ImmPRESS Rabbit HRP (MP-7444, Vector laboratories, CA, USA) or ImmPRESS Mouse HRP (Vector laboratories) was used at 2000 fold dilution.

### Measurement of HR Activity

HeLa cells were seeded on a 3.5 cm dish, and the next day, siRNA was introduced. After 24 hours of culture from the introduction of siRNA, LentiCRISPRv2-ACTB-C1 and pBS-ACTB-200 GFPfr1 (vector for ASHRA measurement) were introduced, and Nicaraven was added at the same time. After further culturing for 48 hours, genomic DNA was purified using Blood Genomic DNA Extraction Mini Kit (Favorgen, Ping-tung, Taiwan). Genome quantitative PCR was performed using Go Taq qPCR master mix (Promega, Madison, WI, USA), measurement was done using CFX96 Touch Real-time PCR detection system (BioRad, Hercules, CA, USA), and knock-in efficiency was calculated by ΔΔCt method. Sequences of a primer for detecting a knock-in allele and a primer for detecting a reference allele are shown below.
For knock-in allyl detection
Forward GTCCTGCTGGAGTTCGTGACCG
Reverse GTGCAATCAAAGTCCTCGGC
For reference allyl detection
Forward AGTTGCGTTACACCCTTTCTTG
Reverse GTGCAATCAAAGTCCTCGGC
Reference paper: NPL 2.

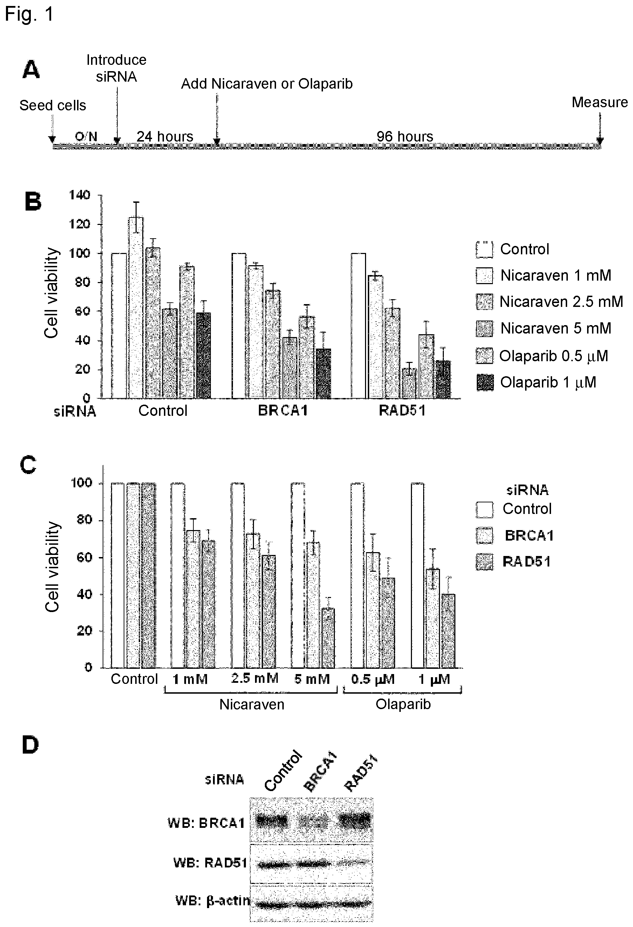

### Example 1 PARP Inhibitory Activity of Nicaraven (Fig. 1)

Fig. 1A shows a protocol of an experiment in which cell viability under Nicaraven or Olaparib treatment on cells having decreased homologous recombination activity was analyzed. siRNA of control, BRCA1 or RAD51 was introduced into HeLa cells, and after 24 hours, Nicaraven or Olaparib was added. After 96 hours of culture, cell viability was measured.

Figs. 1B and 1C show the measurement results of cell viability. Nicaraven treatment decreased cell viability in a concentration-dependent manner. When expression of BRCA1 and RAD51, which are important factors that function in homologous recombination, was suppressed, cell viability further decreased. The decrease in homologous recombination activity caused PARP inhibitory action and synthetic lethality, suggesting that Nicaraven has a PARP inhibitory action. Similarly, treatment with Olaparib, a PARP inhibitor, used as a positive control decreased cell viability in cells in which expression of BRCA1 and RAD51 was suppressed, as compared with the control.

siRNA of control, BRCA1 or RAD51 was introduced into HeLa cells. After 48 hours, a whole cell extract was prepared, and Western blotting was performed with an anti-BRCA1 antibody, an anti-RAD51 antibody, and an anti-β-actin antibody. The results are shown in Fig. 1D. By introduction of siRNA of BRCA1 or RAD51, suppression of each expression was confirmed.

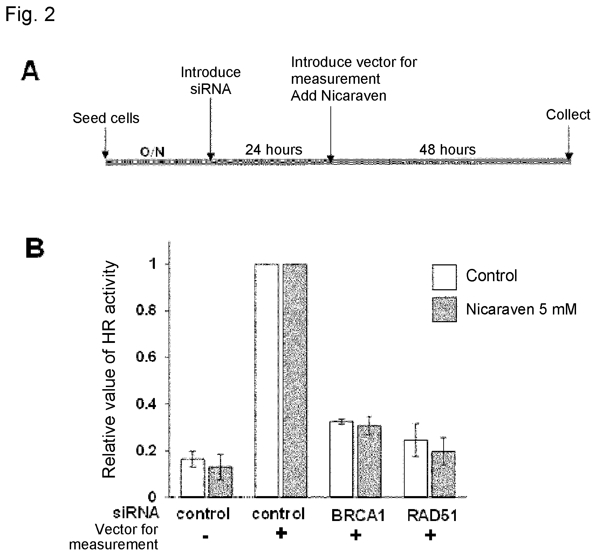

### Example 2 Effect of Nicaraven Treatment on Homologous Recombination Activity (Fig. 2)

Fig. 2A shows a protocol of an experiment in which the effect of Nicaraven treatment on homologous recombination activity was analyzed. siRNA of control, BRCA1 or RAD51 was introduced into HeLa cells. After 24 hours, a vector for measurement of homologous recombination activity was introduced, and Nicaraven was added. After 48 hours of culture, DNA was collected and homologous recombination activity was measured.

Fig. 2B shows the results of measurement of homologous recombination activity. Nicaraven treatment did not affect the homologous recombination ability even in control cells, and cells in which the expression of BRCA1 and RAD51 was suppressed to decrease the homologous recombination activity. The homologous recombination ability was analyzed by Assay for site-specific homologous recombination activity (ASHRA) (Yoshino et al. Scientific Reports 2019, method shown in Fig. 5).

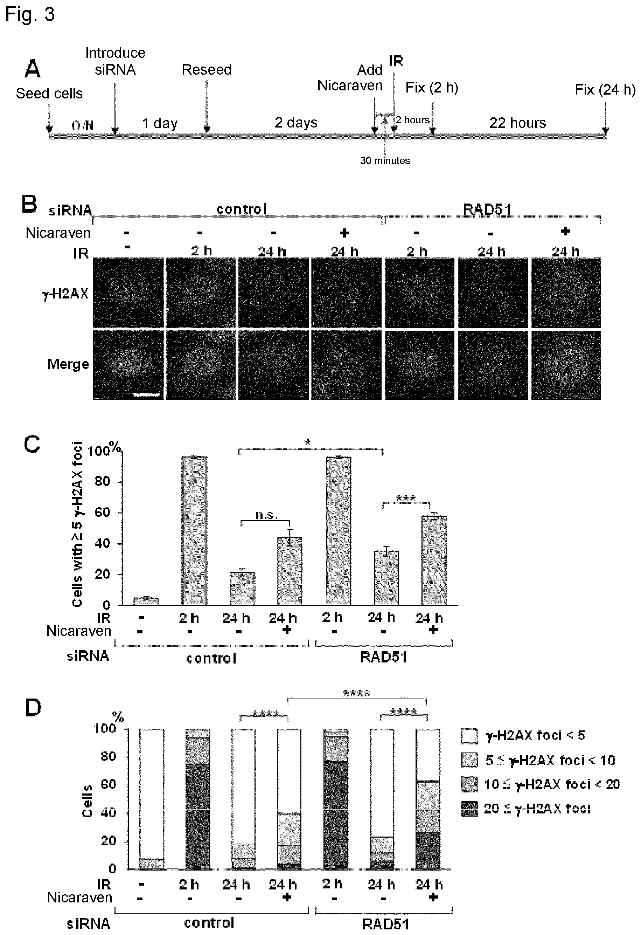

### Example 3 Effect of Nicaraven Treatment on DNA Double Strand Break in DNA Repair Process (Fig. 3)

Fig. 3A shows a protocol of an experiment in which the effect of Nicaraven treatment on repair of DNA damage following ionizing radiation exposure (IR) was analyzed. siRNA of control or RAD51 was introduced into HeLa cells. After cell seeding, Nicaraven was added. After 30 minutes, the cells were irradiated with 1.5 Gy of X-ray, and after 2 hours and 24 hours, the cells were fixed and immunostained with an anti-γ-H2AX antibody.

Fig. 3B shows representative photographs at respective experimental conditions. γ-H2AX is stained green. DNA is shown in blue.

As shown in Fig. 3C, γ-H2AX, a marker of DNA double-strand break, is observed as an intranuclear focus in immunostaining. Cells in which five or more γ-H2AX intranuclear foci were observed were counted, and the ratio was calculated and represented in a graph. Respective cells in which less than 5, 5 or more and less than 10, 10 or more and less than 20, and 20 or more γ-H2AX intranuclear foci were observed were counted, and the ratio was calculated and represented in a graph (Fig. 3D).

After IR, more γ-H2AX intranuclear foci are observed in multiple cells in 2 hours. After 24 hours, DNA double strand break is repaired, reducing the number of cells having intranuclear foci and the number of intranuclear foci per cell. Suppression of expression of RAD51 suppressed DNA damage repair. Nicaraven treatment caused DNA damage to remain in both the control and the state in which the expression of RAD51 was suppressed, but DNA damage remained more remarkably in the state in which the expression of RAD51 was suppressed and the homologous recombination repair activity decreased. It is considered that more DNA damage was formed due to PARP inhibitory action. The measurement of nuclear focus of γ-H2AX 24 hours after X-ray irradiation in this example is a result in a cell having a fast cell cycle. From the above results, it is found that Nicaraven suppresses repair of DNA damage caused by X-ray irradiation by PARP suppressive action in cells having a fast cell cycle such as tumor cells, thereby promoting DNA damage caused by X-ray irradiation.

### Example 4 Effect of Nicaraven Treatment on DNA Double Strand Break Caused by IR (Fig. 4)

Fig. 4A shows a protocol of an experiment in which the effect of Nicaraven treatment on DNA double-strand break caused by IR was analyzed. The day after HeLa cells were seeded, Nicaraven was added, and 30 minutes later, the cells were irradiated with 10 Gy of X-ray. Two hours later, the cells were collected. Cell extracts were adjusted and analyzed with Western Blot. Fig. 4B shows Western Blots with an anti-γ-H2AX antibody, an anti-HistonH3 antibody, and an anti-α-tubulin antibody. The amount of γ-H2AX, a marker of DNA double-strand break, which was increased by IR, was reduced by Nicaraven treatment. It seems that Nicaraven exhibits a function as a radical scavenger. In the measurement 2 hours after the X-ray irradiation in this example, the influence of DNA damage accumulation because of DSB converted from SSB due to the progress of cell cycle is reduced. Accordingly, it is found that Nicaraven exhibits a function as a radical scavenger.

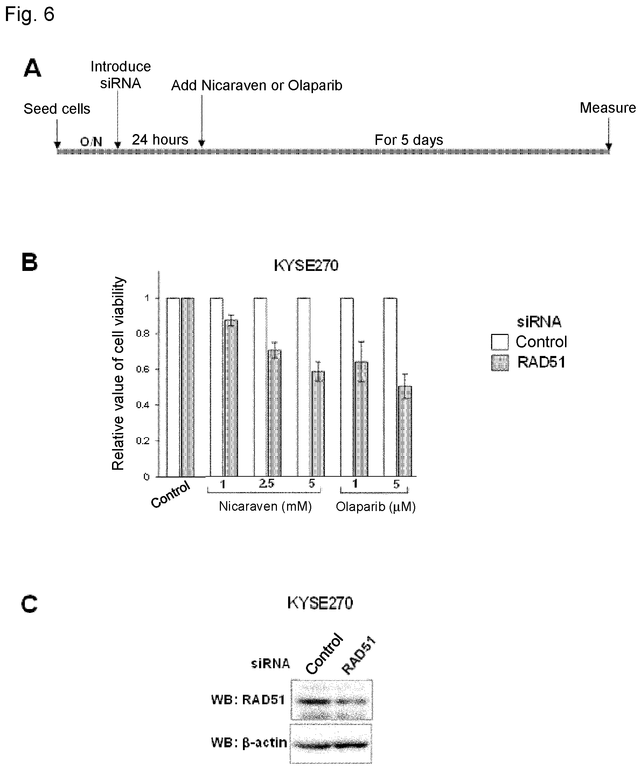

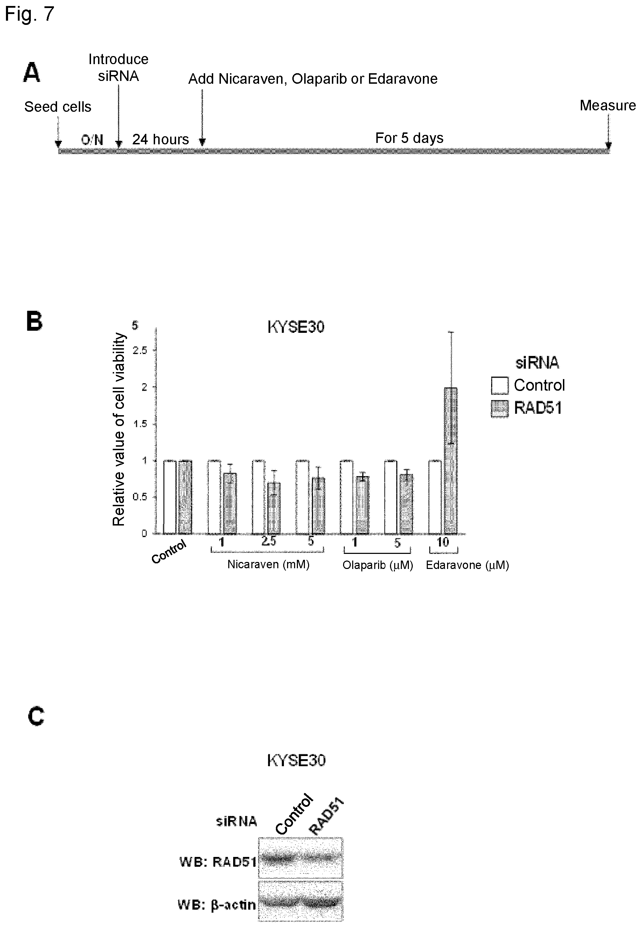

### Example 5 PARP Inhibitory Activity of Nicaraven in Esophageal Cancer Cells (Figs. 6 and 7)

Fig. 6A. Protocol of an experiment in which cell viability under Olaparib, Nicaraven or Edaravone treatment on cells having reduced homologous recombination activity was analyzed. siRNA of control or RAD51 was introduced into esophageal cancer cells KYSE270 cells, and 24 hours later, either Olaparib or Nicaraven was added. After 5 days of culture, cell viability was measured.

Fig. 6B. Measurement results of cell viability in KYSE270 cells. In the Nicaraven treatment, when expression of RAD51, which is an important factor that functions in homologous recombination, is suppressed, cell viability further decreased. The decrease in homologous recombination activity caused PARP inhibitory action and synthetic lethality, suggesting that Nicaraven has a PARP inhibitory action. Similarly, treatment with Olaparib, not a radical scavenger but a PARP inhibitor, used as a positive control decreased cell viability in cells in which expression of RAD51 was suppressed, as compared with the control.

Fig. 6C. siRNA of control or RAD51 was introduced into KYSE270 cells, 3 days later, a whole cell extract was prepared, and Western blotting was performed with an anti-RAD51 antibody and an anti-β-actin antibody. By introduction of siRNA of RAD51, suppression of expression of RAD51 was confirmed.

Fig. 7. Except that KYSE30 cells were used in place of KYSE270, siRNA of RAD51 was introduced, and after 24 hours, any one of Olaparib, Nicaraven and Edaravone was added, the same procedure as described above was performed to analyze cell viability of cells having reduced homologous recombination activity under Nicaraven treatment. As shown in Fig. 7, also in KYSE30 cells, cell viability decreased in cells in which expression of RAD51 was suppressed, as compared with the control.

## Claims

1. A medicine having both radical scavenger activity and PARP inhibitory activity, comprising
Nicaraven as an active ingredient.

2. A medicine for use in suppression of DNA damage caused by irradiation in normal cells by a radical scavenger function and inhibition of repair of DNA damage in malignant tumor (cancer) cells by a PARP suppressive action, comprising
Nicaraven as an active ingredient.

3. The medicine according to claim 1 or 2, being administered to a subject prior to irradiation.
